# EUROPEAN PATENT APPLICATION

(11) **EP 2 428 217 A1**
(43) Date of publication of application: **14.03.2012**
(21) Application number: 11181036.2
(22) Date of filing: 13.09.2011
(51) Int. Cl.: A61K 38/44, A61K 31/202, C12N 9/02, A61K 36/02, A61P 1/16, A61P 1/18, A61P 11/00, A61P 43/00

(54) **Composition based on docosahexaenoic acid (dha) for use in the treatment of cystic fibrosis and non-alcoholic hepatic steatosis**

(30) Priority: 13.09.2010 IT MI20101666
(71) Applicant: DMF Dietetic Metabolic Food S.R.L., 20812 Limbiate (IT)
(72) Inventor: Vicentini, Ultimo Vito, 20182 Limbiate (IT)
(74) Representative: Coppo, Alessandro

(57) **Abstract**

The invention relates to a composition comprising docosahexaenoic acid (DHA), one or more triglycerides of straight-chain saturated fatty acids having from 6 to 12 carbon atoms and vegetable superoxide dismutase (SOD). The composition according to the invention is suitable in the treatment of cystic fibrosis and/or non-alcoholic hepatic steatosis, in particular in paediatrics.

## Description

### FIELD OF THE INVENTION

This invention relates to a composition based on docosahexaenoic acid (DHA) in the treatment of cystic fibrosis and steatosis, in particular in pediatrics.

### PRIOR ART

Docosahexaenoic acid is an essential fatty acid of the omega-3 acid series. The eicosanoids derived from the long-chain polyunsaturated fatty acids (LCPUFAs) of the omega-3 series have an anti-inflammatory effect, as opposed to those derived from omega-6 which have a pro-inflammatory action.

DHA therefore plays a crucial role in generating the type-3 prostaglandins and series-5 leukotrienes that are involved in the anti-inflammatory response, by inhibiting pro-inflammatory cytokines and nitric oxide production.

The intravenous administration of omega-3 derivatives, such as DHA, has been associated with a reduction in inflammatory diseases, such as psoriasis, through changes in the generation of inflammatory eicosanoids (Masser P, Grimm H, Grimmer F. N-3 fatty acids in psoriasis. Br J Nutr 2002. 87 (suppl. 1): 577- 582). The diseases with inflammatory causes also include rare diseases such as cystic fibrosis (CF) and non-alcoholic hepatic steatosis or non-alcoholic fatty liver disease (NAFLD).

The recurrent respiratory infections during the course of cystic fibrosis result in a chronic state of inflammation of the airways, and it is the prolongation of the inflammatory process over the years, with frequent exacerbations, that eventually causes damage to the bronchial and pulmonary tissue and consequently a decrease in respiratory function. To combat the invasion of pathogens, the cells release many substances into the tissue including some that are strong oxidants, so-called reactive oxygen species (ROS) which, over the long term, damage the tissues.

For some time, therefore, the belief has become established that patients suffering with CF, in addition to receiving antibiotic therapy, should also be given treatment to counteract the ROS. Attention has been focused on various antioxidants such as glutathione and DHA (Corti et al., Vitamin C supply to bronchial epithelial cells linked to glutathione availability in ELF - a role for secreted gamma-glutamyltransferase? J. Cyst. Fibrosis 7: 174-8, 2008) .

Cystic fibrosis has been associated with a lower level of linoleic acid and DHA in tissues and plasma, as the metabolism of these cells for omega-3 was accelerated. Supplementing the diet with DHA can correct this metabolic defect. DHA is well tolerated in the long term, as well as having an anti-inflammatory role on pulmonary tissue.

However, to date cystic fibrosis is still treated with antibiotics, since the administration of DHA alone is not sufficient to ensure a satisfactory outcome in the medium term.

The term "hepatic steatosis" indicates an increase in the content of fats, generally triglycerides, within the cells forming the liver tissue. This condition is referred to in more simple terms as "fatty liver". The formation of steatosis is closely related to the role played by the liver in lipid metabolism and is caused by the imbalance between the intake of fatty acids into the liver and their breakdown, resulting in an accumulation of triglycerides in the hepatocytes in the form of droplets (microvesicular steatosis) or a single large drop (macrovesicular steatosis). This process occurs in the presence of factors that together promote lipogenesis instead of lipolysis, for example the increased uptake and intrahepatic synthesis of fatty acids and their reduced elimination. NAFLD may be associated with an inflammatory and fibrotic condition similar to that of alcoholic hepatopathy in terms of its anatomo-pathological clinical picture. With regard to patients with non-alcoholic hepatic steatosis, there is in fact a distinction of great importance to be drawn between "simple" steatosis and "steatohepatitis" (NASH), which represents an advanced and more severe stage. In simple steatosis, the presence of fat is the only abnormality, and is associated with mild inflammation. It is an essentially benign condition, but the liver remains more vulnerable to causes of liver damage. NASH involves the presence of steatosis, inflammation and signs of hepatocytic damage and/or fibrosis. Compared with alcoholic hepatitis, the steatosis is more pronounced and appears in the form of a large vacuole in the hepatocyte. The inflammatory infiltrate is evident at the intralobular level and is characterised by neutrophils and mononuclear cells. Tissue damage is present in the form of hepatocytic ballooning and necrosis. The evolution towards decompensation occurs through the stimulation of a tissue repair process which, in the form of fibrogenesis, leads gradually to the anatomical and functional changes of cirrhosis. There are forms of non-alcoholic steatosis secondary to other causes such as genetic diseases, infections, and exposure to toxic substances; insulin resistance is one of the most important predictors of the development of NAFLD. NAFLD is considered to be an integral part of "metabolic syndrome" or "insulin resistance syndrome" characterised by impaired glucose tolerance and resistance to insulin, excess body weight with a prevalence of abdominal fat deposits, high blood pressure and altered lipid and glucose metabolism leading to the presence of type 2 diabetes and cardiovascular diseases associated with atherosclerosis.

For some years, a correlation has been noted between non-alcoholic fatty liver disease (NAFLD), obesity and depletion of polyunsaturated omega-3 hepatic fatty acids. The change in the parameters indicating oxidative stress shows a moderate progression towards a pro-oxidative state of the liver and thus towards steatosis, which deteriorates over time into steatohepatitis. Oxidative stress plays a dual role in NAFLD by exacerbating steatosis with high peroxidation of LCPUFAs and by disrupting the metabolism of fatty acids that promotes the progression to steatohepatitis (Videla LA, Rodrigo R, Araya J, Poniachik J. Oxidative stress and depletion of hepatic long- chain polyunsaturated fatty acids may contribute to nonalcoholic fatty liver disease. Free Radic Biol Med. 2004 Nov 1; 37 (9): 1499-5079).

In NAFLD, there is a constant accumulation of triglycerides inside the hepatocytes, through which all of the protective mechanisms of the cell are unable to prevent, and the rate of hepatocyte death exceeds their ability to regenerate. The overall response involves myofibroblasts, which generate an excessive myofibrillar matrix, which in turn stimulates the expansion of the hepatocyte progenitors. The progenitor cells secrete chemokines and a wide range of pro-inflammatory molecules that lead to a distortion of the cellular architecture of the liver.

As mentioned above, patients with NAFLD have such strong oxidative stress that it contributes to the reduction of LCPUFAs, as well as a worsening of the disease. It has been reported that DHA plays a crucial role in reducing body fat in obese patients with hepatic steatosis or non-alcoholic fatty liver precisely because it limits the initial inflammatory mechanism. It is most useful if administered in children, since it prevents the formation of excess fat that will lead to the child being overweight.

The various studies show the presence of obesity in the 90-95% of patients with NAFLD, and in the paediatric population more than one child in four is overweight. The pathogenic factors responsible for the progression of steatosis are not yet clear, but it has been determined that the excessive accumulation of fat increases the vulnerability of hepatocytes to the potential harmful effects of metabolic stress caused by oxidants, cytokines and viral infections.

The disease is often asymptomatic, but there are cases of abdominal pain, malaise and asthenia. The close epidemiological relationship with obesity has led to a fundamental evaluation of the effects of weight loss, which also has beneficial effects on insulin. Lipotoxicity, oxidative stress, cytokines and other pro-inflammatory mediators in the pathogenesis of steatosis have suggested the administration of antioxidants as a possible therapeutic approach (Marra F, S. La steatosi epatica non alcolica. II Nostro Fegato. 2007; no. 30).

Cystic fibrosis has also been reported in childhood. Cystic fibrosis (CF) is an autosomal recessive disorder traced to a mutation on chromosome 7, which causes an abnormality of the transmembrane conductance regulator gene. As a result, excessive transepithelial secretion of salts causes a diagnostic increase in chloride and sodium in sweat. 80% of affected children have pancreatic exocrine insufficiency (PEI). The symptoms in evidence are steatorrhoea and a deficiency of fat-soluble vitamins (A, D, E and K) as well as depletion of mineral salts such as zinc and selenium.

It is also known that the addition of protein supplements to the diet of infant patients can provide up to 4 g / kg / day of protein, while it is not necessary to restrict the intake of fats that are usually well tolerated up to 40g / day.

In children who suffer from this disease, the energy requirement is high and intakes of food are low as a result of their pulmonary disease, malabsorption, chronic infections, state of weakness and asthenia. There has been promotion of feeding by nasogastric tubes using elementary formulas or intact nutrients associated with pancreatic enzyme supplements for use in children and adolescents.

It is precisely in patients with fat malabsorption, as in the case of CF, that the essential fatty acid deficiency is more evident clinically. The symptoms of deficiency of essential fatty acids appear when linoleic acid accounts for less than 1 % of the caloric intake.

Human milk has 3% to 7% of its calories composed of linoleic acid, plus a significant amount that comes from the food eaten by the mother. Most commercial infant formulas contain more than 10% fat in the form of linoleic acid and a certain amount of linolenic acid. Cow's milk contains only 1%, a lesser amount since the daily requirement is 2.7%.

In children with steatorrhoea, decreased levels of prostaglandins returned to normal after administration of supplements rich in linoleic acid and calories.

To date, there are no commercially available products to treat cystic fibrosis and steatosis as an alternative to drug treatments associated with dietary supplements, particularly in paediatrics.

There is therefore a need not only to provide a single product to treat such diseases, but also to provide at the same time calories and amounts of essential fats suitable for the growth of a sick child.

One of the aims of this invention is therefore to provide a composition for the treatment of cystic fibrosis and/or steatosis, particularly in paediatrics.

### SUMMARY OF THE INVENTION

In a first aspect the present invention concerns a composition comprising
- docosahexaenoic acid (DHA);
- One or more triglycerides of straight-chain saturated fatty acids having from 6 to 12 carbon atoms;
- A vegetable superoxide dismutase (SOD).

The inventors of the present invention have found that the association of docosahexaenoic acid with one or more triglycerides with saturated fatty acids with a medium chain having a length of 6 to 12 carbon atoms and a superoxide dismutase (SOD) results in a synergistic activity in the treatment and/or prophylaxis of cystic fibrosis and or steatosis, in particular in pediatric patients.

In certain embodiments the composition of the invention is a drug or a natural health product.

In certain embodiments the composition of the invention is a dietary product or a dietary supplement. The term dietary supplement means a preparation or composition intended to supplement the diet and provide nutrients, such as lipids, anti-oxidants molecules, and other ingredients that may be missing or may not be consumed in sufficient quantities in a person's diet with particular reference to diet of persons affected by cystic fibrosis or steatosis.

In a second aspect the composition as above defined is provided for the use as a medicament.

In a third aspect, the invention relates to the aforementioned composition for use in the treatment of cystic fibrosis.

In a fourth aspect, the present invention relates to the above composition for use in the treatment of steatosis.

In a fifth aspect, the present invention provides for the use of the above composition in the treatment of cystic fibrosis and steatosis in pediatric patients.

### DETAILED DESCRIPTION OF THE INVENTION

According to a first aspect the invention relates to a pharmaceutical composition or a dietary supplement comprising the synergistic association of
i) docosahexaenoic acid (DHA) or an oil containing DHA;
ii) One or more triglycerides of straight-chain saturated fatty acids having from 6 to 12 carbon atoms,
iii) A vegetable superoxide dismutase (SOD).
and a physiologically acceptable carrier and/or excipient. Typically, the active ingredients i) to iii) are contained in the composition of the invention in an amount which is effective in the treatment or prevention of metabolic disorders such as cystic fibrosis or steatosis.

Docosahexaenoic acid (22:6 n-3 or DHA) is an essential fatty acid of the omega-3 series (PUFA). Its chemical structure is a chain comprising 22 carbon atoms, along which 6 double cis bonds are distributed; the first of these links the third and fourth carbon atoms from the methyl portion (terminal), hence the name w3.

In accordance with certain embodiments of the invention the DHA source is an algae or a microalgae in particular of the genus *Schizochytrium* which is a natural food source for fish.

In accordance with certain embodiments the composition of the invention comprises an oil containing docosahexaenoic acid (DHA) which preferably is extracted from Schizochytrium Sp. The extraction of the oil from the algae may be made by conventional extraction techniques.

In certain embodiments, the medium-chain triglycerides (MCTs) used in the composition of the invention are triglycerides whose glycerol molecule are linked to straight-chain saturated fatty acids with 6-12 carbon atoms, for example such as C6:0 caproic acid, C8:0 caprylic acid, C10:0 capric acid and C12:0 lauric acid.

In certain embodiments the composition of the invention comprises one or more medium-chain triglycerides (MCTs) in the quantity of between 10% and 30%, typically of 22% to 28%, for example around 25.5% by weight in respect of the weight of the total composition.

The third ingredient of the composition of the invention encompasses any enzymes belonging to the family of superoxide dismutase (SOD). These enzymes belong to the class of oxidoreductases and transforms the superoxide into less harmful molecules such as oxygen and hydrogen peroxide.

In certain embodiments the SOD is of vegetal origin, and preferably is obtained from *Cucumis melo.*

In accordance with another aspect of the invention, the inventors have found that when the SOD enzyme is formulated in a gastro-resistant form, the degradation of the enzyme in the gastric environment is reduced and the synergistic activity with the other active ingredients of the composition is improved.

In certain embodiments the composition of the invention comprises SOD in the quantity of between 0.2% and 2%, typically of 0.5% to 1.6%, for example around 1.1 % by weight of the total composition.

In certain embodiments the SOD of the composition of the invention is in the form of a gastro-resistant formulation to prevent deactivation of the enzyme by the low pH of the gastric environment.

In certain embodiment the SOD enzyme is coated with a lipid which typically is solid at environmental temperature. A suitable oil can be palm oil.

In certain embodiments the SOD enzyme is encapsulated for example in microspheres of modified starch and covered with a protective coating for example of a gum. The gastro resistant formulation of SOD permits the enzyme to pass through the gastric barrier without being deactivated.

In certain embodiments the SOD enzyme is made gastro-resistant by dispersing the SOD in a physiologically acceptable liquid for example a melon juice which is then adsorbed into microspheres for example made of modified starch and optionally then coated with a gum such as Arabic gum.

The suspension of the SOD enzyme in the liquid, makes the enzyme resistant to thermal stress and acids preserving its bioactivity during the passage in the gastric environment.

In certain embodiments, the composition of the invention further includes one or more additional components, such as aromas, fillers and thickeners.

In certain embodiments the aromas that can be added are selected from fruit flavors, and even more preferably hazelnut and citrus aromas.

In certain embodiments the thickeners of the composition include anhydrous colloidal silica and glyceryl monostearate.

In certain embodiments the composition of the invention comprises oil of algal origin with 35% DHA in the quantity of between 55% and 85%, more preferably 60% to 65%, and even more preferably around 63.3% by weight of the total composition.

The composition according to the invention can be administered orally.

In certain embodiments the composition comprises a physiologically or pharmaceutically acceptable carrier, excipient or diluent. The physiologically or pharmaceutically acceptable carrier, excipient or diluent may be chosen based on the intended route of administration, mainly the oral routes.

In certain embodiments the composition of the invention suitable for oral administration is in the form of tablets, capsules, suspensions, solutions or emulsions, syrup and jam.

In certain embodiments the composition according to the invention is administered in capsule form, for example in the form of soft gelatin capsules.

The use of the composition of the invention avails of certain advantages. The composition utilises natural products which are effective and safe for environmental and the user.

The specific association of the ingredients i, ii, iii of the composition of the invention combines an high antinflammatory activity with an antioxidative effect by inhibiting both pro-inflammatory cytokines and nitric oxide production. This effect provides a benefit in the treatment of rare and chronic diseases with inflammatory causes.

Advantageously, the invention has demonstrated efficacy in the treatment of cystic fibrosis, steatosis, non-alcoholic hepatic steatosis or non-alcoholic fatty liver disease (NAFLD). The natural origins of the ingredients i) to iii) makes the composition specifically indicated for pediatric applications.

In these diseases, it is preferably administered as a "health food for special medical purposes".

Therefore, in certain aspects the invention relates to the above compositions for use in the treatment or prophylaxis of cystic fibrosis or/and steatosis, in particular in children for pediatric use.

The composition of the invention may be administered in a "dietetic or antinflammatory acceptable amount", this being an amount sufficient to at least partially reduce the general inflammation of the human body. Alternatively, the composition may be administered to a subject in need of treatment to a "therapeutically effective amount" this being an amount sufficient to show benefit to the subject. In particular the benefit may be the amelioration of at least a symptom associated with the condition to be treated or the prevention or partial inhibition of the onset of at least one symptom associated with the condition. The severity and or time of onset of at least one symptom may be reduced with the administration of the composition.

Typically, the actual amount and the rate and time course of administration of the composition will depend on the nature and severity of the condition to be treated. Prescription of treatment such as decisions on dosage and frequency of administration is within the responsibility of and at discretion of general practitioners and other medical doctors and typically resemble the dosage of DHA-based nutritional supplements/pharmaceutical products available on the market.

In certain embodiments the composition, in the form of single dosage units, is administered by the oral route, one to four times a day.

The following examples are provided merely for illustrating the present invention and are not to be intended as limiting the scope of protection resulting from the appended claims.

### EXAMPLE 1

A composition in the form of capsules containing DHA, triglycerides of straight-chain saturated fatty acids having from 6 to 12 carbon atoms, a vegetable superoxide dismutase (SOD) as active ingredients was prepared.

### 1) Preparation of the core (nucleus).

The following ingredients were used in the amounts indicated:

| Ingredient | mg per capsule |
|---|---|
| oil from *Schizochytrium Sp* with a DHA content of 35% by weight | 285 |
| triglycerides (C₆-C₁₂) | 115 |
| glyceryl monostearate | 30 |
| hazelnut aroma | 10 |
| vegetable SOD (from *Cucumis melo*) | 5 |
| colloidal silica | 5 |
| **Total** | **450** |

The triglycerides and glyceryl monostearate were transferred to a turbo-emulsifier, with vacuum aspiration. The mixture was heated to 60±5°C, preserving the vacuum until a homogeneous solution was obtained.

The algal oil (DHA) was added during agitation and the mixture was cooled under vacuum to 25-30°C.

The colloidal silica was added and the mass was agitated under vacuum until a homogeneous suspension was obtained.

Then, under atmospheric pressure, the SOD and hazelnut aroma were added, and the product obtained was agitated under vacuum for 30 minutes. The preparation was also de-aerated. The preparation was then placed in a stainless-steel container with a drain valve for feeding directly into the encapsulator and the product was incubated at 30°C.

The container with the mass to be encapsulated was then sent to the encapsulation department for the next stage of encapsulation.

### 2) Preparation of the capsule shell

The following ingredients were used in the amounts indicated:

| Capsule shell ingredient | mg/capsule |
|---|---|
| food-grade gelatin of bovine origin | 166.55 |
| glycerol-based firming agent | 133.3 |
| glycine | 4.3 |
| silicates as an anticaking agent E555 | 3.045 |
| titanium oxide as a colouring E171 | 1.950 |
| iron oxide as a flowability agent E172 | 0.230 |
| TOTAL | 309.375 |

### PREPARATION OF THE BASE GLYCOGELATIN

The glycerol and purified water were added to the suitable stainless-steel container, equipped with a heating jacket with water circulation, a mixer with counter-rotating blades and a system for operating in a vacuum.

The mass was heated to 60°C under constant agitation and operating in a vacuum.

Gelatin was added and the mass was agitated in a vacuum until it was completely fused, it was then discharged under pressure into a suitable stainless-steel container kept at a temperature of 55°C.

The following colorings were then added to the mass: Titanium dioxide E171; black iron oxide E172; aluminum and potassium silicate E 555. The mass was agitated with a special Ystral agitator until a homogeneous colour was achieved.

### ENCAPSULATION

The containers to be encapsulated and those of the base glycogelatin were checked according to the processing cards available in the department.

The containers were then placed on the encapsulator machine and production was started. The mass of hot gelatin fed 2 suitable meters on the machine forming two films of a determined and constant thickness on two air-cooled rollers. The two films passed through two capsule forming cylinders which rotate in a concentric movement, above which was a particular heated wedge, known as the injector segment. The mixture to be encapsulated was then fed directly to the metering pump equipped with precision syringes which, running with a powered reciprocating motion, through the injector segment, formed the capsules in the cavities of the two forming cylinders.

The sealed and cut capsules fell underneath the forming cylinders on the conveyor belt that conveyed them to rotating pre-drying racks from which, after about two hours, they were transferred to special trays (flats). The processing conditions were maintained at 20-25°C with 10-30% relative humidity.

### Weight checking during processing

Every 30 minutes, a number of capsules was sampled corresponding to a row of cavities in the forming cylinder. We proceeded to perform a single weighing to determine the weight of the contents. The weight was recorded on the relevant cards. In this way, the precision of dosing was guaranteed within the limits of +/-5%.

### DRYING

After the formation of the capsules, the drying phase with rolling was started immediately. The capsules were then unloaded onto flat trays and transferred to a suitable drying area. Once dried, the capsules were transferred to the visual inspection and bulk packaging site.

### VISUAL INSPECTION

A visual inspection and mechanical sorting was performed with an automatic selector of each capsule by the operators. Defective units were therefore removed. We then proceeded with the operation of inserting the capsules into polythene bags for food use and then into cartons for storage while awaiting transfer to packaging (blister packs / bottles) if required. Capsules with the following nutritional values were obtained.

| | | Per capsule |
|---|---|---|
| Energy value | Kcal | 5.069 |
| | Kj | 21.19 |
| Proteins | g | 0.167 |
| Carbohydrates | g | 0.133 |
| Fats | g | 0.430 |
| -saturated | g | 0.165 |
| -monounsaturated | g | 0.021 |
| -polyunsaturated | g | 0.147 |
| -- DHA | mg | 100.000 |
| SOD | mg | 5.000 |

### EXAMPLE 2

Nutritional supplement for adults in encapsulated powder.

SOD: in microgranules, 1mg= 14U.I. The enzyme was coated by vegetal fats derived from palm oil.

| Ingredients | Mg per capsula |
|---|---|
| oil from Schizochytrium Sp with a DHA content of 35% by weight | 600 |
| Triglycerides with medium chain | 100 |
| glyceryl monostearate | 60 |
| Hazelnut Aroma | 15 |
| Anydrous colloidal silica | 10 |
| SOD from Cucumis melo | 5 |
| Total | 790 |

### EXAMPLE 3

### Formulation for administration to adult people

SOD is encapsulated in microspheres of modified starch and covered by a protective coating made of Arabic gum. Titration 1 mg= 1U.I. oily formulation

| Ingredients | Mg per capsule |
|---|---|
| oil from *Schizochytrium Sp* with a DHA content of 35% by weight | 600 |
| triglycerides (C₆-C₁₂) | 100 |
| glyceryl monostearate | 60 |
| Hazelnut aroma | 15 |
| Anydrous colloidal silica | 10 |
| SOD from Cucumis melo | 5 |
| Total | 790 |

### EXAMPLE 4

### Dietetic product in powder form for children (pediatric use)

SOD: in microgranules, 1 mg= 14U.I. The enzyme was coated by fats derived from vegetal oil.

| Ingredients | Mg per capsula |
|---|---|
| Fish Oil containing DHA 40% | 285 |
| Triglycerides with medium chain | 115 |
| glyceryl monostearate | 30 |
| Aroma | 10 |
| Anydrous colloidal silica | 5 |
| SOD from Cucumis melo | 5 |
| Total | 450 |

## Claims

1. A composition comprising:
i) docosaesaenoic acid (DHA),
ii) one or more triglycerides of saturated fatty acids comprising from 6 to 12 carbon atoms,
iii) dismutase superoxide (SOD) and
a physiologically acceptable carrier or excipient.

2. Composition according to claim 1, **characterised in that** the docosaesaenoic acid (DHA) is dispersed in an oil extracted from algae.

3. Composition according to claim 2 **characterised in that** the algae belong to the species Schizochytrium Sp.

4. Composition according to claim 1 **characterised in that** the DHA is dispersed in an oil extracted from Schizochytrium Sp and said oil is in an amount from 55% to 85% by weight.

5. Composition according to anyone of claims 1 to 4, **characterised in that** the dismutase superoxide (SOD) is formulated as a gastro-resistant form.

6. Composition according to claim 5 **characterised in that** the gastro-resistant form comprises SOD coated by a lipid which is solid at environmental temperature.

7. Composition according to claim 6 **characterised in that** the lipid which is solid at environmental temperature is palm oil.

8. Composition according to claim 1 to 5 **characterised in that** the SOD is encapsulated in microspheres of modified starch.

9. Composition of claim 8 **characterised in that** said microspheres of modified starch are coated by a gum.

10. Composition according to anyone of claims from 1 to 9, **characterised in that** it further contains a component selected from fragrances, fillers and thickeners.

11. Composition according to claim 10, **characterised in that** the thickeners are selected from the group consisting of colloidal anhydrous silica and glycerol monostearate.

12. Composition according to anyone of claims from 1 to 11, wherein the composition is in the form of tablets, capsules, suspensions or emulsions, syrup jam or marmalade.

13. Composition according to anyone of claims from 1 to 9 **characterised in that** it is a dietary supplement or a natural health product.

14. Composition according to anyone of claims from 1 to 13 for use in the treatment of cystic fibrosis, especially in paediatrics.

15. Composition according to anyone of claims from 1 to 13 for use in the treatment of non alcoholic fatty liver disease (NAFLD) or hepatic steatosis, especially in paediatrics.
